(19) Europäisches Patentamt
European Patent Office
Office européen des brevets

(11) Publication number : **0 399 183 B1**

(12) # EUROPEAN PATENT SPECIFICATION

(45) Date of publication of patent specification : **28.06.95 Bulletin 95/26**

(51) Int. Cl.[6] : **G03C 7/34,** // C07C309/69, C07C311/31, C07C317/28, C07C317/32

(21) Application number : **90106271.1**

(22) Date of filing : **02.04.90**

(54) **Photographic recording material containing a cyan dye-forming coupler.**

(30) Priority : **07.04.89 US 335166**

(43) Date of publication of application : **28.11.90 Bulletin 90/48**

(45) Publication of the grant of the patent : **28.06.95 Bulletin 95/26**

(84) Designated Contracting States : **DE FR GB**

(56) References cited :
**EP-A- 0 159 913**
**EP-A- 0 271 324**
**EP-A- 0 349 331**
**US-A- 4 009 035**

(73) Proprietor : **EASTMAN KODAK COMPANY**
**343 State Street**
**Rochester, New York 14650-2201 (US)**

(72) Inventor : **Merkel, Paul Barret, c/o EASTMAN KODAK COMPANY**
**Patent Department,**
**343 State Street**
**Rochester, New York 14650 (US)**
Inventor : **Hoke, David, c/o EASTMAN KODAK COMPANY**
**Patent Department,**
**343 State Street**
**Rochester, New York 14650 (US)**

(74) Representative : **Brandes, Jürgen, Dr.rer.nat. et al**
**Schweigerstrasse 2**
**D-81541 München (DE)**

EP 0 399 183 B1

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to a photographic recording material containing a phenolic cyan dye-forming coupler. More particularly, this invention relates to a photographic recording material containing a coupler which is used to obtain a cyan dye for color photography which coupler is typically a phenol or a naphthol which yields an azomethine dye upon coupling with oxidized aromatic primary amino color developing agent.

Known cyan couplers, including those of U. S. Patent 4,333,999, have highly desirable properties in that they can provide dyes of excellent purity and hues which are shifted bathochromically to the long wavelength red absorption region. Although such couplers have been widely used, further improvements in coupler reactivity and enhanced dye absorption continue to be sought. For example, it has been difficult to obtain, with the same coupler, a dye having both high density and high coupling effectiveness. A cyan coupler yielding enhanced dye density would allow use of less image coupler in a layer. This would provide enhanced cost savings as well as image sharpness improvements. Coupling effectiveness is measured by comparing the gamma or Dmax of the resulting dye image sensitometric test curve with that of a control coupler under identical conditions.

The presence of sulfone ($-SO_2-$) groups in ballast moieties of cyan coupler compounds has been described in various publications. These publications include Japanese Patent Publication Numbers 105644/1984 (priority of December 10, 1982), 111643/1984 and 111644/1984 (both having priorities of December 17, 1982). Couplers having sulfone groups in the ballast moiety are also disclosed in U. S. Patents 4,609,619 and 4,775,616 and cyan couplers having modified sulfone groups, that is groups comprising $-OSO_2-$ or $-NHSO_2-$ moieties, are described in U. S. Patent 4,849,328.

A highly desirable property of cyan dye-forming couplers is an ability to provide acceptable dye density while at the same time providing dyes having the desired hue and saturation.

The present invention provides a photographic recording material comprising a support and a photosensitive silver halide emulsion characterized in that the emulsion has associated therewith a cyan dye-forming coupler compound having the structural formula:

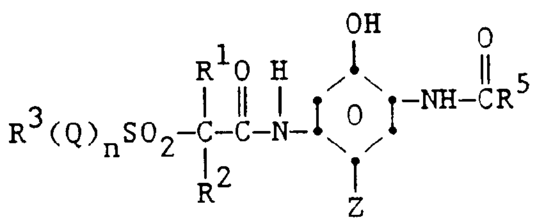

wherein:

$R^1$ is an unsubstituted or a substituted, straight or branched chain alkyl group having from 1 to about 20 carbon atoms, an unsubstituted or a substituted cycloalkyl group having from 3 to about 8 carbon atoms in the ring or an unsubstituted or a substituted aryl group;

$R^2$ is as defined for $R^1$ or is hydrogen;

$R^3$ is an unsubstituted or a substituted alkyl group having from 1 to about 24 carbon atoms, an unsubstituted or a substituted cycloalkyl group having from 3 to about 8 carbon atoms in the ring, an unsubstituted or a substituted aryl group having from 6 to about 24 carbon atoms or an unsubstituted or a substituted heterocyclic group having from 3 to about 8 atoms in the heterocyclic ring, wherein the hetero ring atoms can be nitrogen, oxygen, or sulfur;

with the proviso that when $R^3$ is a primary alkyl group, $R^1$ must contain at least 2 carbon atoms;

Q is oxo or $-NR^4-$;

$R^4$ is hydrogen; an unsubstituted or a substituted alkyl group having from 1 to about 24 carbon atoms; an unsubstituted or a substituted cycloalkyl group having from 3 to about 8 carbon atoms in the ring; or an unsubstituted or a substituted aryl group having from 6 to about 24 carbon atoms;

$R^5$ is an unsubstituted or a substituted linear or branched alkyl group having from 1 to about 32 carbon atoms;

n is 0 or 1; and

Z is hydrogen or a coupling off group,

with the proviso that at least one of the $R^1$, $R^2$, $R^3$, $R^4$, $R^5$ or Z groups is of such size and configuration as to render the coupler compound substantially nondiffusible in the layer of a photographic recording material in which it is coated.

The specifically described combination in a phenolic coupler of (a) a sulfone or a modified sulfone-containing ballast group in the 5- position and a 2- position substituted acylamino group provides couplers yielding high dye density while preserving other desirable dye properties. These results are surprising and could not have been predicted from the body of knowledge available before the investigations leading to this invention were carried out.

In preferred coupler compounds of this invention $R^1$ is alkyl having 1 to about 14 carbon atoms and $R^2$ is hydrogen. In particularly preferred coupler compounds $R^1$ is alkyl of 1 to about 4 carbon atoms and $R^2$ is hydrogen.

When the $R^3$, $R^4$ or $R^5$ groups are substituted such substituents may include alkyl, aryl, alkoxy, aryloxy, alkylthio, arylthio, hydroxy, halogen, alkoxycarbonyl, aryloxycarbonyl, carboxy, acyl, acyloxy, carbonamido, carbamoyl, alkylsulfonyl, arylsulfonyl, sulfonamido and sulfamoyl groups wherein the alkyl and aryl substituents, and the alkyl and aryl moieties of the alkoxy, aryloxy, alkylthio, arylthio, alkoxycarbonyl, arylcarbonyl, acyl, acyloxy, carbonamido, carbamoyl, alkylsulfonyl, arylsulfonyl, sulfonamido and sulfamoyl substituents can contain, respectively, from 1 to about 30 carbon atoms and from 6 to about 30 carbon atoms and can be further substituted with such substituents.

The $R^5$ alkyl groups can be substituted with one or more of halogen atoms, preferably chloro or fluoro, alkoxy groups, alkylthio groups, arylthio groups, alkylsulfonyl groups, arylsulfonyl groups, sulfonamido groups, acylamino groups, alkyloxycarbonyl groups, aryloxycarbonyl groups, carbamoyl groups, alkylcarbonyloxy groups, arylcarbonyloxy groups, carboxyl groups, cyano groups and hydroxyl groups.

Coupling off groups defined by Z are well known to those skilled in the art. Such groups can determine the equivalency of the coupler i.e., whether it is a 2-quivalent coupler or a 4-equivalent coupler. Such groups can also modify the reactivity of the coupler or can advantageously affect the layer in which the coupler is coated, or other layers in a photographic recording material, by performing, after release from the coupler, such functions as development inhibition, bleach inhibition, bleach acceleration and color correction.

Representative classes of coupling-off groups include alkoxy, aryloxy, heteroyloxy, sulfonyloxy, acyloxy, acyl, heterocyclyl, sulfonamido, phosphonyloxy and arylazo. These coupling-off groups are described in the art, for example, in U. S. Patent Nos. 2,455,169, 3,227,551, 3,432,521, 3,476,563, 3,617,291, 3,880,661, 4,052,212 and 4,134,766; and in U. K. Patents and published applications Nos. 1,466,728, 1,531,927, 1,533,039, 2,006,755A and 2,017,704A.

Examples of preferred coupling-off groups which can be represented by Z are:

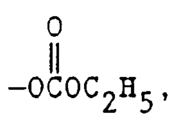 , 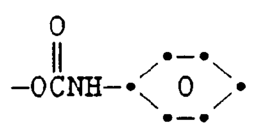 , 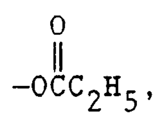 , 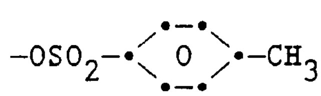 ,

$-OCH_3$, $-OC_6H_5$, $-OCH_2CONHCH_2CH_2OH$,
$-OCH_2CONHCH_2CH_2OCH_3$, $-OCH_2CONHCH_2CH_2OCOCH_3$,

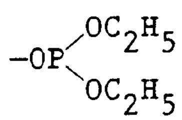 , 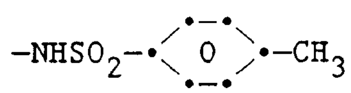 ,

$-OCH_2CH_2NHSO_2CH_3$ and

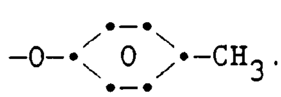 .

Especially preferred Z groups are hydrogen and

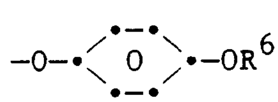

where $R^6$ is an alkyl or an alkoxy group having from 1 to about 10 carbon atoms, such as

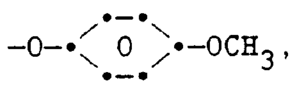 and 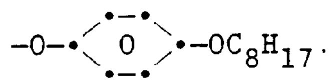.

Specific coupler compounds of this invention are shown below in Table 1 with reference to the following structural formula:

Table 1

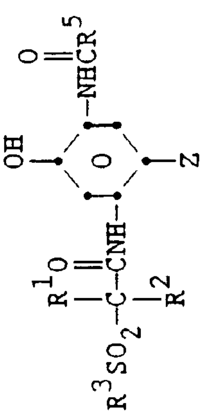

| Coupler Compound | $R^1$ | $R^2$ | $R^3$ | $R^5$ | $Z^*$ |
|---|---|---|---|---|---|
| 1 | $-C_{10}H_{23}$ | H | $-C_{18}H_{37}$ | $-C_3F_7$ | A |
| 2 | $-C_{14}H_{29}$ | H | $-CH_3$ | $-C_3F_7$ | H |
| 3 | $-C_{10}H_{21}$ | H | 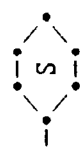 | $-C_3F_7$ | H |
| 4 | $-C_2H_5$ | H | $C_{16}H_{33}$ | $-C_3F_7$ | H |
| 5 | $-C_2H_5$ | H | $C_{16}H_{33}$ | $-CH_2CF_3$ | H |
| 6 | $-C_2H_5$ | H | $C_{16}H_{33}$ | $-CH_2CN$ | H |
| 7 | $-C_2H_5$ | H | $C_{16}H_{33}$ | $-CF_3$ | H |

* A = 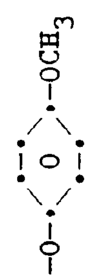

B = 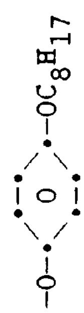

Further specific coupler compounds useful in this invention are shown below in Table 2 with reference to the following structural formula:

Table 2

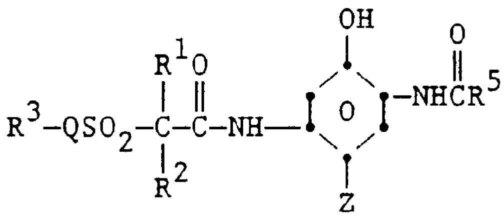

Table 2

| Coupler Compound | $R^1$ | $R^2$ | $R^3$ | Q | $R^5$ | $Z^*$ |
|---|---|---|---|---|---|---|
| 8 | $-C_{14}H_{29}$ | H | $-CH_3$ | -O- | $-C_3F_7$ | A |
| 9 | $-C_{10}H_{21}$ | H | 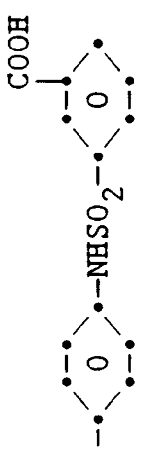 | -NH- | $-C_3F_7$ | H |
| 10 | $-C_{10}H_{21}$ | H | 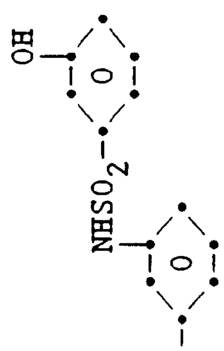 | -NH- | $-C_3F_7$ | A |

* A = 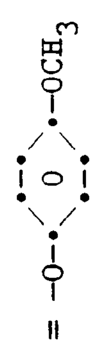

Coupler compounds of this invention can be prepared by reacting alkyl acid halides with an appropriate aminophenol, such as 2-amino-5-nitro-phenol or 2-amino-4-chloro-5-nitro phenol to form the 2-carbonamido coupler moiety compounds. The nitro group can then be reduced to an amine and a separately prepared ballast moiety can be attached thereto by conventional procedures. Two-equivalent couplers can be prepared by known techniques, for example, by substitution of a 4-chloro group on the starting phenol. Details of such preparations are noted below relative to specific coupler compounds identified in Tables I and II.

SYNTHESIS Example 1

Coupler Compound No. 4 was prepared as follows:

A Preparation of phenolic coupler moiety:

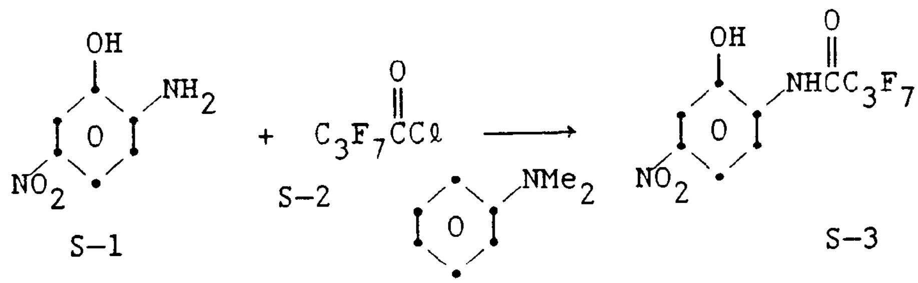

S-1 S-2 S-3

S-3

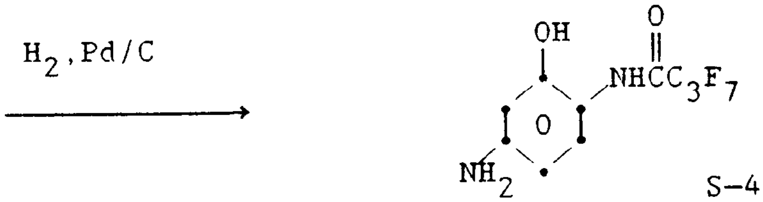


S-4

To a stirred solution of 30.8g (0.20 mol) of 2-amino-5-nitro phenol (S-1) and 53.2g (0.44 mole) of N,N-dimethylaniline in 500ml ethyl acetate is added 51g (0.22 mole) of heptafluoro butyryl chloride (S-2). After 3 hours stirring at ambient temperature, the reaction mixture is washed twice with 10% HCl, dried and concentrated to a solid. Crystallization from acetonitrile yielded 58.5g of S-3.

A solution of 6g (0.0171 mole) of nitro compound S-3 in 150ml ethyl acetate was shaken for 3 hours with 1.5g 10% palladium on carbon catalyst and 1ml acetic acid under 276kPa (40 lb) hydrogen pressure to provide the aminophenol S-4.

B. Preparation of the ballast acid chloride:

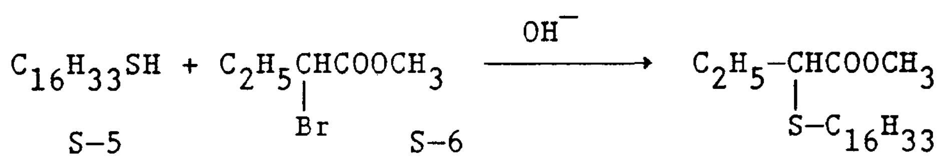


S-5 S-6 S-7

S-7

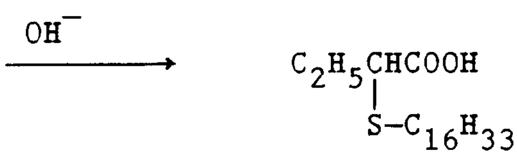


S-8

S-8

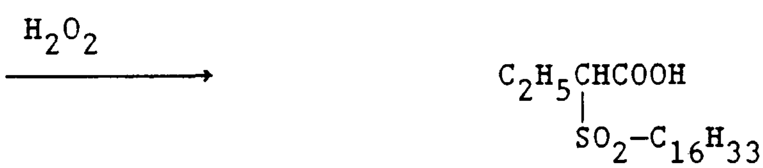


S-9

S-9

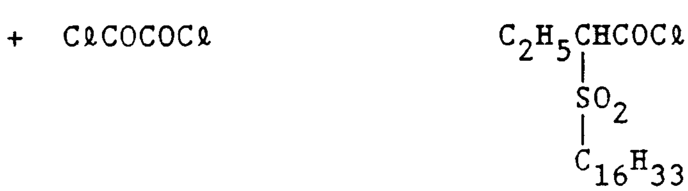

S-10

To a well stirred solution of 258.4g (1mol) n-hexadecyl mercaptan (S-5 and 217g (1.2 mole) methyl alpha-bromobutyrate (S-6) in 500 ml ethanol was added, under nitrogen, a solution of 44g (1.1 mol) sodium hydroxide in 300ml water. After 1 hour a solution of 80g (2 mol) sodium hydroxide in 1 liter tetrahydrofuran and 750ml methanol was added and stirring continued 2 hours to hydrolyze the S-7 ester to the S-8 acid sodium salt. This salt, which precipitated on concentration of the reaction mixture, was suspended in 4.5 liters dilute hydrochloric acid and stirred 1 hour to yield 350g moist white solid S-8 acid.

A solution of 1g tungstic acid in aqueous sodium hydroxide was made slightly acidic by titration with acetic acid and then added at 30 degree C to a solution of 290g (0.84 mol) S-8 acid in 1.15 liter acetic acid. To this solution was added dropwise with stirring over a 30 minute period 210g (1.85 mole) of 30% hydrogen peroxide solution. After 2 hours additional stirring while heating with a steam bath, the reaction mixture was allowed to cool to 30 degree C and a white crystalline product was isolated by filtration. Washing with ligroin and acetonitrile yielded 233g S-9 acid.

7.1g (0.0188 mol) of the S-9 acid in 150ml dichloromethane was treated with 3.2ml (0.0377 mole) oxalyl chloride and 10 drops dimethylformamide, stirred for 1.5 hours, and concentrated to yield 7.3g ballast acid chloride S-10 as white solid.

C. Synthesis of Coupler Compound No. 4:

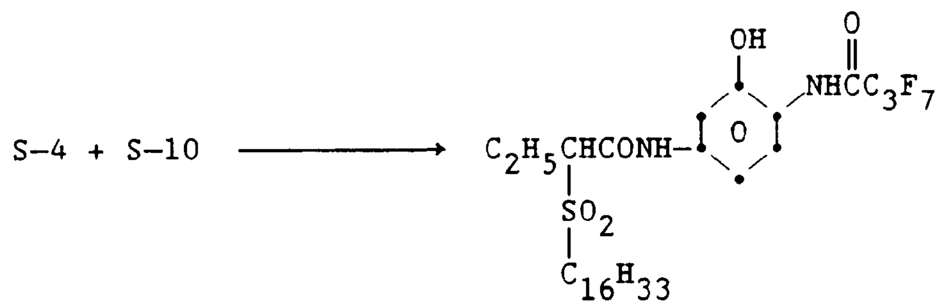


Coupler Compound No. 4

To a stirred solution of 5.5g (0.0171 mole) of the S-4 amine and 6.2g (0.0543 mole) N,N-dimethylaniline in 300ml ethyl acetate is added 7.3g (0.01885 mole) of S-10 ballast acid chloride. After stirring for 1 hour, the reaction mixture is washed three times with 10% HCl, dried and concentrated to a solid. Crystallization from acetonitrile gave 10.8g white solid. Mass spec and elemental analysis confirm product structure as that of Coupler Compound No. 4.

The cyan dye-forming couplers of this invention can be used in the ways and for the purposes that cyan dye-forming couplers are used in the photographic art. Typically, the couplers are incorporated in silver halide emulsions and the emulsions coated on a support to form a photographic element. Alternatively, the couplers can be incorporated in photographic elements adjacent the silver halide emulsion where, during development, the coupler will be in reactive association with development products such as oxidized color developing agent.

The cyan dye-forming couplers described herein can be combined with development inhibitor releasing compounds having the structure:

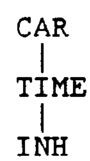

where CAR is a carrier moiety, TIME is a timing group and INH is a development inhibitor moiety. Such combination enhances the sharpness of cyan images obtained from the described couplers.

The INH development inhibitor moiety is more fully described in co-pending U. S. Patent Application Serial No. 213,415 filed June 30, 1988.

As used herein, the term "in reactive association" signifies that the coupler is in the silver halide emulsion layer or in an adjacent location where, during processing, it is capable of reacting with silver halide development products.

The photographic elements can be either single color or multicolor elements. In a multicolor element, the

cyan dye-forming coupler of this invention is usually associated with a red-sensitive emulsion, although it could be associated with an unsensitized emulsion or an emulsion sensitized to a different region of the spectrum. Multicolor elements contain dye image-forming units sensitive to each of the three primary regions of the spectrum. Each unit can be comprised of a single emulsion layer or of multiple emulsion layers sensitive to a given region of the spectrum. The layers of the element, including the layers of the image-forming units, can be arranged in various orders as known in the art.

A typical multicolor photographic element comprises a support bearing a cyan dye image-forming unit comprising of at least one red-sensitive silver halide emulsion layer having associated therewith at least one cyan dye-forming coupler, at least one of the cyan dye-forming couplers being a coupler of this invention, a magenta dye image-forming unit comprising at least one green-sensitive silver halide emulsion layer having associated therewith at least one magenta dye-forming coupler and a yellow dye image-forming unit comprising at least one blue-sensitive silver halide emulsion layer having associated therewith at least one yellow dye-forming coupler. The element can contain additional layers, such as filter layers, interlayers, overcoat layers, subbing layers, and the like.

In the following discussion of suitable materials for use in the elements of this invention, reference will be made to Research Disclosure, December 1978, Item 17643, published by Kenneth Mason Publications, Ltd., The Old Harbourmaster's, 8 North Street, Emsworth, Hampshire PO10 7DD, ENGLAND. This publication will be identified hereafter by the term "Research Disclosure."

The silver halide emulsions employed in the elements of this invention can be either negative-working or positive-working. Suitable emulsions and their preparation are described in Research Disclosure Section I and II and the publications cited therein. Tabular photographic silver halide grains are also useful. Such tabular grain silver halide is described in, for example, U. S. Patent 4,434,226 and in Research Disclosure, January 1983, Item No. 22534. Suitable vehicles for the emulsion layers and other layers of elements of this invention are described in Research Disclosure Item 17643, Section IX and the publications cited therein.

In addition to the couplers described herein the elements of this invention can include additional couplers as described in Research Disclosure Section VII, paragraphs D, E, F and G and the publications cited therein. These additional couplers can be incorporated as described in Research Disclosure Section VII, paragraph C and the publications cited therein.

The photographic elements of this invention can contain brighteners (Research Disclosure Section V), antifoggants and stabilizers (Research Disclosure Section VI), antistain agents and image dye stabilizers (Research Disclosure Section VII, paragraphs I and J), light absorbing and scattering materials (Research Disclosure Section VIII), hardeners (Research Disclosure Section XI), plasticizers and lubricants (Research Disclosure Section XII), antistatic agents (Research Disclosure Section XIII), matting agents (Research Disclosure Section XVI) and development modifiers (Research Disclosure Section XXI).

The photographic elements can be coated on a variety of supports as described in Research Disclosure Section XVII and the references described therein.

Photographic elements can be exposed to actinic radiation, typically in the visible region of the spectrum, to form a latent image as described in Research Disclosure Section XVIII and then processed to form a visible dye image as described in Research Disclosure Section XIX. Processing to form a visible dye image includes the step of contacting the element with a color developing agent to reduce developable silver halide and oxidize the color developing agent. Oxidized color developing agent in turn reacts with the coupler to yield a dye.

Preferred color developing agents ae p-phenylene diamines. Especially preferred are 4-amino-3-methyl-N,N-diethylaniline hydrochloride, 4-amino-3-methyl-N-ethyl-N-β-(methanesulfonamido)ethylaniline sulfate hydrate, 4-amino-3-methyl-N-ethyl-N-β-hydroxyethylaniline sulfate, 4-amino-3-β-(methanesulfonamido)ethyl-N,N-diethylaniline hydrochloride and 4-amino-N-ethyl-N-(2-methoxyethyl)-m-toluidine di-p-toluene sulfonic acid.

With negative working silver halide this processing step leads to a negative image. To obtain a positive (or reversal) image, this step can be preceded by development with a non-chromogenic developing agent to develop exposed silver halide, but not form dye, and then uniform fogging of the element to render unexposed silver halide developable. Alternatively, a direct positive emulsion can be employed to obtain a positive image.

Development is followed by the conventional steps of bleaching, fixing, or bleach-fixing to remove silver and silver halide, washing and drying.

In the following examples, a measure of each coupler's coupling effectiveness is represented by G which is the ratio of its photographic dye image gamma (the slope of the sensitometric curve) to that of the analogous nonsulfone Control Couplers. Such normalization of the data compensates for coating and processing variations by relating the performance of each test coupler as described herein to that of a control coupler coated and processed at the same time and in the same manner.

Processing and testing procedures were kept constant. Particularly useful couplers provided dye images

with G > 1.00, and Dmax>Dmax of the control.

Example 1

Dispersions of a coupler of the invention (No. 4), Comparative Coupler C and respective analogous nonsulfone couplers (A) and (B) were prepared. The coupler solvent dibutylphthalate was used at a 1:0.5 coupler:coupler solvent weight ratio. Each coupler was coated at 1,56 mmoles/$m^2$ (0.15 mmoles/$ft^2$) with 874 mg/$m^2$ (84 mg/$ft^2$) of silver as a medium-large grain, polydisperse AgBrI (6.5 mole % I) emulsion. The coatings were exposed and subjected to an E-6 process with and without citrazinic acid (CZA) in the color developer at 0.57 g/l. Results are shown below:

| Coupler | G (with CZA) | G (no CZA) | Dmax (with CZA) | Dmax (no CZA) | λmax(nm) |
|---|---|---|---|---|---|
| A (Control) | 1.00 | 1.00 | 1.54 | 2.02 | 656 |
| 4 | 1.68 | 1.32 | 2.64 | 3.24 | 646 |
| B (Control) | 1.00 | 1.00 | 2.68 | 3.34 | 641 |
| C (Comparative) | 0.11 | 0.50 | 1.04 | 1.29 | 622 |

It is evident that the use of a sulfone-containing ballast dramatically improves G values and Dmax values for the 2-alkylcarbonamido coupler No. 4, but leads to inferior G and Dmax values for the comparative 2-arylcarbonamido Coupler C.

Control Coupler A

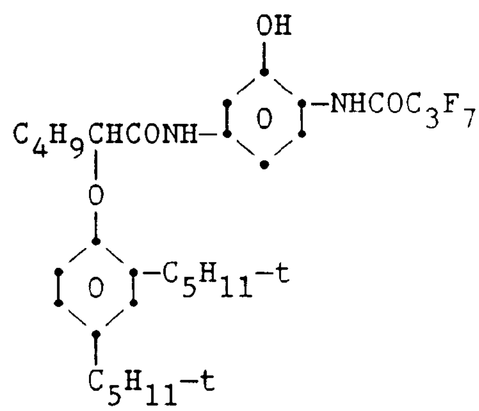

Control Coupler B

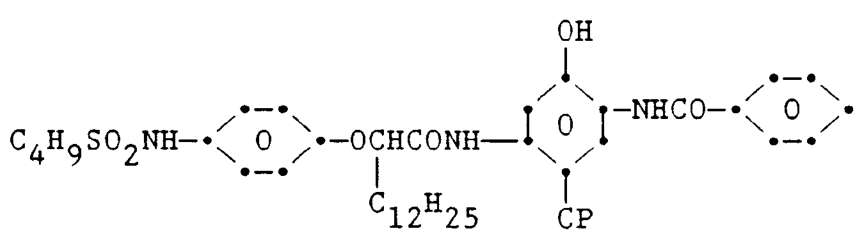

Comparative Coupler C

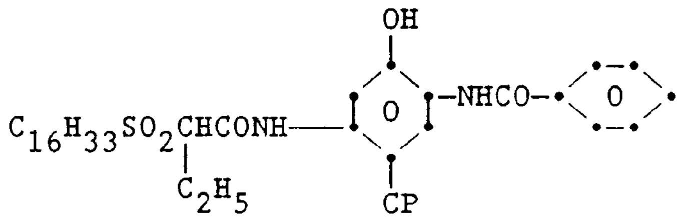

**Claims**

1. A photographic recording material comprising a support and a photosensitive silver halide emulsion characterized in that the emulsion has associated therewith a cyan dye-forming coupler compound having the structural formula:

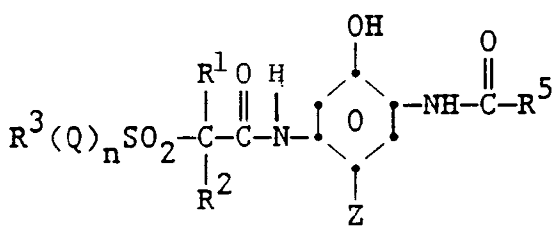

wherein:

$R^1$ is an unsubstituted or a substituted, straight or branched chain alkyl group having from 1 to 20 carbon atoms, an unsubstituted or a substituted cycloalkyl group having from 3 to 8 carbon atoms in the ring or an unsubstituted or a substituted aryl group;

$R^2$ is as defined for $R^1$ or is hydrogen;

$R^3$ is an unsubstituted or a substituted alkyl group having from 1 to 24 carbon atoms, an unsubstituted or substituted cycloalkyl group having from 3 to 8 carbon atoms in the ring, an unsubstituted or a substituted aryl group having from 6 to 24 carbon atoms or an unsubstituted or a substituted heterocyclic group having from 3 to 8 atoms in the heterocyclic ring, wherein the hetero ring atoms can be nitrogen, oxygen, or sulfur;

with the proviso that when $R^3$ is a primary alkyl group $R^1$ must contain at least 2 carbon atoms;

Q is -O- or $-NR^4-$;

$R^4$ is hydrogen; an unsubstituted or a substituted alkyl group having from 1 to 24 carbon atoms; an unsubstituted or a substituted cycloalkyl group having from 3 to 8 carbon atoms in the ring; or an unsubstituted or a substituted aryl group having from 6 to 24 carbon atoms;

$R^5$ is an unsubstituted or a substituted linear or branched chain alkyl group having from 1 to 32 carbon atoms;

n is 0 or 1; and

Z is hydrogen or a coupling off group;

with the proviso that at least one of the $R^1$, $R^2$, $R^3$, $R^4$, $R^5$ or Z groups is of such size and configuration as to render the coupler compound substantially nondiffusible in the layer of the photographic recording material in which it is coated.

2. The photographic material of claim 1 wherein Q is $-NR^4-$ or 0.

3. The photographic element of claim 1 wherein $R^1$ is alkyl of from 1 to 14 carbon atoms and $R^2$ is hydrogen.

4. The photographic material of claim 4 wherein $R^1$ is alkyl of from 1 to 4 carbon atoms and $R^2$ is hydrogen.

5. The photographic material of claim 2 wherein $R^4$ is hydrogen and $R^5$ is an alkyl substituted with one or

more halogen atoms.

6. The photographic material of claim 1 wherein Z is hydrogen or

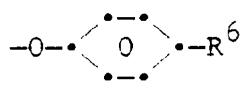

where $R^6$ is an alkyl or an alkoxy group having from 1 to 10 carbon atoms.

7. The photographic material of claim 1 wherein the cyan dye-forming coupler compound has the structural formula:

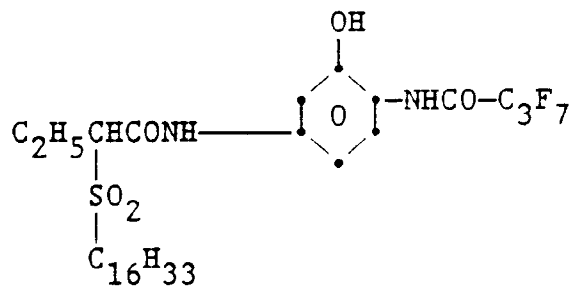

## Patentansprüche

1. Photographisches Aufzeichnungsmaterial mit einem Träger und einer photosensitiven Silberhalogenidemulsion, dadurch gekennzeichnet, daß der Emulsion eine einen blaugrünen Farbstoff bildende Kupplerverbindung zugeordnet ist, die der Strukturformel entspricht:

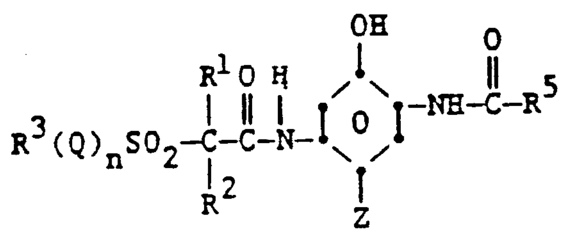

worin bedeuten:
$R^1$ eine unsubstituierte oder eine substituierte geradkettige oder verzweigtkettige Alkylgruppe mit 1 bis 20 Kohlenstoffatomen, eine unsubstituierte oder eine substituierte Cycloalkylgruppe mit 3 bis 8 Kohlenstoffatomen im Ring oder eine unsubstituierte oder eine substituierte Arylgruppe;
$R^2$ ein Gruppe wie für $R^1$ angegeben oder ein Wasserstoffatom;
$R^3$ eine unsubstituierte oder eine substituierte Alkylgruppe mit 1 bis 24 Kohlenstoffatomen, eine unsubstituierte oder substituierte Cycloalkylgruppe mit 3 bis 8 Kohlenstoffatomen im Ring, eine unsubstituierte oder eine substituierte Arylgruppe mit 6 bis 24 Kohlenstoffatomen oder eine unsubstituierte oder eine substituierte heterocyclische Gruppe mit 3 bis 8 Atomen im heterocyclischen Ring, wobei die Heteroringatome aus Stickstoff, Sauerstoff oder Schwefel bestehen können;
wobei gilt, daß wenn $R^3$ eine primäre Alkylgruppe ist, $R^1$ mindestens 2 Kohlenstoffatome enthalten muß;
Q gleich -O- oder $-NR^4-$;
$R^4$ ein Wasserstoffatom; eine unsubstituierte oder eine substituierte Alkylgruppe mit 1 bis 24 Kohlenstoffatomen; eine unsubstituierte oder eine substituierte Cycloalkylgruppe mit 3 bis 8 Kohlenstoffatomen im Ring; oder eine unsubstituierte oder eine substituierte Arylgruppe mit 6 bis 24 Kohlenstoffatomen;
$R^5$ eine unsubstituierte oder eine substituierte lineare oder verzweigtkettige Alkylgruppe mit 1 bis 32 Kohlenstoffatomen;
n gleich 0 oder 1: und

Z ein Wasserstoffatom oder eine abkuppelnde Gruppe;
wobei gilt, daß mindestens eine der Gruppen $R^1$, $R^2$, $R^3$, $R^4$, $R^5$ oder Z eine solche Größe und Konfiguration aufweist, daß sie die Kupplerverbindung in der Schicht des photographischen Aufzeichnungsmaterials, in der sie aufgetragen worden ist, praktisch nicht-diffundierend macht.

**2.** Photographisches Material nach Anspruch 1, in dem Q für $-NR^4-$ oder O steht.

**3.** Photographisches Element nach Anspruch 1, in dem $R^1$ für Alkyl mit 1 bis 14 Kohlenstoffatomen steht und $R^2$ für Wasserstoff.

**4.** Photographisches Material nach Anspruch 3, in dem $R^1$ eine Alkylgruppe mit 1 bis 4 Kohlenstoffatomen ist und $R^2$ für Wasserstoff steht.

**5.** Photographisches Material nach Anspruch 2, in dem $R^4$ für Wasserstoff steht und $R^5$ für eine Alkylgruppe, die durch ein oder mehrere Halogenatome substituiert ist.

**6.** Photographisches Material nach Anspruch 1, in dem Z für Wasserstoff steht oder für

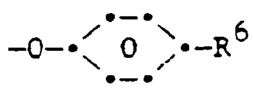 ,

worin $R^6$ eine Alkyl- oder eine Alkoxygruppe mit 1 bis 10 Kohlenstoffatomen ist.

**7.** Photographisches Material nach Anspruch 1, in dem die einen blaugrünen Farbstoff bildende Kupplerverbindung die folgende Strukturformel hat:

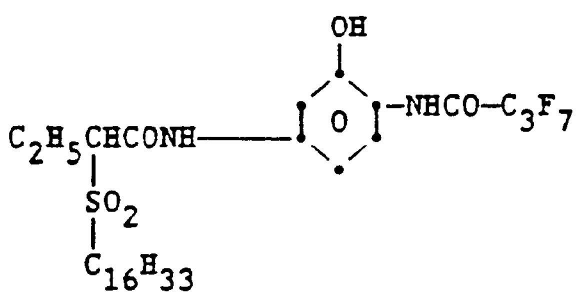

## Revendications

**1.** Produit d'enregistrement photographique comprenant un support et une émulsion aux halogénures d'argent photosensible, caractérisé en ce qu'un coupleur formateur de colorant cyan représenté par la formule structurale :

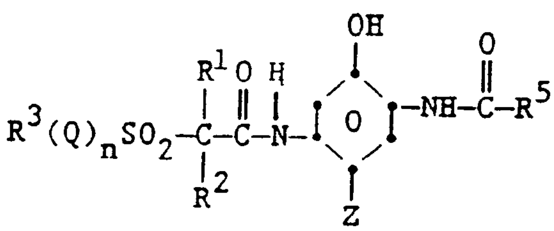

est associé à l'émulsion, et dans lequel :
$R^1$ est un groupe alkyle substitué ou non, à chaîne droite ou ramifiée de 1 à 20 atomes de carbone, un groupe cycloalkyle substitué ou non de 3 à 8 atomes de carbone dans le cycle ou un groupe aryle substitué ou non ;

$R^2$ est tel que défini pour $R^1$ ou est l'hydrogène ;
$R^3$ est un groupe alkyle substitué ou non de 1 à 24 atomes de carbone, un groupe cycloalkyle substitué ou non de 3 à 8 atomes de carbone dans le cycle, ou un groupe aryle substitué ou non de 6 à 24 atomes de carbone ou un hétérocycle substitué ou non de 3 à 8 atomes de carbone dans l'hétérocycle, dans lequel les atomes de l'hétérocycle peuvent être l'azote, l'oxygène ou le soufre ;
avec la condition que lorsque $R^3$ est un groupe alkyle primaire, $R^1$ doit contenir au moins deux atomes de carbone ;
Q est -O- ou $-NR^4-$ ;
$R^4$ est l'hydrogène ; un groupe alkyle substitué ou non de 1 à 24 atomes de carbone ; un groupe cycloalkyle substitué ou non de 3 à 8 atomes de carbone dans le cycle ou un groupe aryle substitué ou non de 6 à 24 atomes de carbone ;
$R^5$ est un groupe alkyle ssubstitué ou non à chaîne linéaire ou ramifiée de 1 à 32 atomes de carbone ;
n est 0 ou 1 ; et
Z est l'hydrogène ou un groupe se séparant au couplage ;
avec la condition qu'au moins un des groupes $R^1$, $R^2$, $R^3$, $R^4$, $R^5$ ou Z soit d'une taille et d'une configuration telles qu'il rende le coupleur pratiquement non diffusible dans la couche du produit d'enregistrement photographique dans laquelle il est appliqué.

2. Produit photographique selon la revendication 1, dans lequel Q est $-NR^4-$ ou O.

3. Elément photographique selon la revendication 1, dans lequel $R^1$ est un groupe alkyle de 1 à 14 atomes de carbone et $R^2$ est l'hydrogène.

4. Produit photographique selon la revendication 4, dans lequel $R^1$ est un groupe alkyle de 1 à 4 atomes de carbone et $R^2$ est l'hydrogène.

5. Produit photographique selon la revendication 2, dans lequel $R^4$ est l'hydrogène et $R^5$ est un groupe alkyle substitué par un ou plusieurs atomes d'halogène.

6. Produit photographique selon la revendication 1, dans lequel Z est l'hydrogène ou

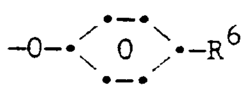 ,

où $R^6$ est un groupe alkyle ou alkoxy de 1 à 10 atomes de carbone.

7. Produit photographique selon la revendication 1, dans lequel le coupleur formateur de colorant cyan est représenté par la formule structurale :